# FASCICULE DE BREVET EUROPEEN

(11) **EP 1 041 060 B1**
(45) Date de publication et mention de la délivrance du brevet: **04.06.2003**
(21) Numéro de dépôt: 00400574.0
(22) Date de dépôt: 02.03.2000
(51) Int. Cl.: C07C 11/14, C07C 11/22

(54) **Procédé de production de méthylacétylène et de propadiène**
Verfahren zur Herstellung von Methylacetylen und Propadien
Process for the preparation of methylacetylene and propadiene

(30) Priorité: 31.03.1999 FR 9904124
(43) Date de publication de la demande: 04.10.2000
(73) Titulaire: Institut Francais du Petrole, 92852 Rueil-Malmaison Cedex (FR)
(72) Inventeur: Busson, Christian, 69260 Charbonnière (FR)

(56) Documents cités:
- FR-A- 1 389 102
- FR-A- 2 732 014

## Description

L'invention concerne un procédé de production de méthylacétylène et de propadiène par conversion thermique d'une charge renfermant au moins un hydrocarbure ayant au moins trois atomes de carbone dans sa molécule.

La synthèse du méthylacétylène et du propadiène est connu de l'homme du métier. Elle s'effectue le plus souvent par pyrolyse de propylène, et/ou d'isobutène ou à partir de propane, de butane, de but-1-ène ou d'un mélange d'isomères de but-2-ènes. La demande de brevet FR 2 732 014 de la demanderesse décrit un procédé de conversion thermique d'hydrocarbures aliphatiques saturés ou insaturés en hydrocarbures acétyléniques. Ledit procédé s'applique particulièrement à la production d'acétylène ou de méthylacétylène mais il ne décrit pas la formation de propadiène.

La demande de brevet US 5 321 191 de la demanderesse décrit un procédé de pyrolyse thermique d'hydrocarbures à au moins deux atomes de carbone. Le procédé est destiné à la production d'oléfines légères, et plus particulièrement de l'éthylène et du propylène.

Les demandes de brevet de la demanderesse EP 0 323 287, US 5 160 501, et US 5 365 005 décrivent un procédé de conversion thermique du méthane en hydrocarbures de poids moléculaire plus élevé, ainsi qu'un réacteur pour la mise en oeuvre du procédé.

La demande de brevet US 5 554 347 de la demanderesse décrit un dispositif comprenant un réacteur pour la mise en oeuvre de réactions comme la pyrolyse. Ledit réacteur comporte des moyens d'échange de chaleur qui sont alimentés en gaz ou en mélange de gaz.

Les demandes de brevets précédemment cités utilisent un réacteur comprenant des moyens de chauffage, qui sont entourés de gaines en matériau céramique. Lesdits moyens sont alimentés soit en énergie électrique, soit au gaz, de façon à chauffer la charge, en vue de la convertir. Ledit réacteur est similaire à celui employé dans la présente demande.

On connaît également un procédé de production d'allène et de méthyl-acétylène, décrit dans le brevet français FR-1 389 102; ce procédé met en oeuvre un craquage en présence d'acide bromhydrique HBr, à des températures de l'ordre de 1000 °C. Cette utilisation d'acide à très haute température conduit à une mise en oeuvre relativement délicate et coûteuse.
Un des avantages de l'invention est de pouvoir réaliser la conversion thermique d'une charge tout en contrôlant la température lors de la décomposition, ce qui n'est actuellement pas réalisable avec les réacteurs de vapocraquages classiques. Le procédé selon l'invention permet de définir des profils de température. Il définit une zone de chauffage découpée en trois parties dans lesquelles s'effectuent des montées contrôlées en températures.

L'invention concerne un procédé de production de méthylacétylène et de propadiène dans une zone de réaction, de forme allongée selon une direction (un axe), comprenant une zone de chauffage et une zone de refroidissement faisant suite à ladite zone de chauffage, dans lequel on fait circuler, dans la zone de chauffage, un mélange gazeux renfermant au moins un hydrocarbure à au moins trois atomes de carbone et au moins un diluent, à une pression absolue supérieure à la pression atmosphérique selon une direction d'écoulement sensiblement parallèle à la direction (à l'axe) de la zone de chauffage, ledit procédé étant caractérisé en ce que la zone de chauffage comprend au moins une zone de préchauffage dans laquelle la température dudit mélange gazeux augmente d'environ 500 à 1200°C par longueur de zone de chauffage, au moins une zone de pyrolyse de la charge dans laquelle la température monte d'environ 200 à 500°C par longueur de zone de chauffage et au moins une zone de formation de méthylacétylène - propadiène dans laquelle la température monte d'environ 700 à 1500°C par longueur de zone de chauffage, les produits formés à l'extrémité de la zone de chauffage étant refroidis dans la zone de refroidissement puis recueillis à l'extrémité de la zone réactionnelle.

Dans une forme particulière du procédé selon l'invention, la zone de chauffage comporte au moins deux rangées sensiblement parallèles à l'axe séparées par une cloison non étanche en matériau réfractaire entre deux rangées successives, chaque rangée comprenant une pluralité de moyens de chauffage disposés en au moins une nappe d'éléments chauffants et entourés de gaines en matériau céramique sensiblement parallèles entre elles et sensiblement perpendiculaires à l'axe de la zone de chauffage.

Les charges hydrocarbonées utilisées dans le cadre de la présente invention comprennent des hydrocarbures ayant au moins trois atomes de carbone dans leur molécule. A titre d'exemple non limitatif, il s'agit d'hydrocarbures aliphatiques saturés tels que le propane et les mélanges d'alcanes (LPG) ou d'hydrocarbures insaturés tels que le propylène et les butènes, les mélanges d'alcanes et d'alcènes tels que le propane et le propylène, les coupes C₃, C₄ et C₅ produites par craquage catalytique en lit fluide, par vapocraquage, par déshydrogénation d'alcanes, par isomérisation d'oléfines ou par dimérisation.
De préférence, la charge contient essentiellement du propylène et/ou du propane et provient du vapocraquage.

Dans les conditions normales de pressions et de températures, les charges sont des mélanges gazeux, qui renferment également au moins un diluent. Ledit diluent est habituellement choisi dans le groupe formé par la vapeur d'eau et l'azote. De préférence, on utilise de la vapeur d'eau. Le rapport pondéral du diluent à la charge hydrocarbonée est habituellement d'environ 0,1:1 à 5:1, de préférence d'environ 0,5:1 à 2,5:1. Le mélange gazeux, avant d'être introduit dans la zone de chauffage, est préalablement chauffé à une température habituellement comprise entre environ 100 et 650°C. Il est ensuite introduit parallèlement à l'axe de la zone de chauffage.

La zone de chauffage est formée d'au moins une zone de préchauffage, d'au moins une zone de pyrolyse et d'au moins une zone de formation de méthylacétylène - propadiène. Elle est le plus souvent chauffée par des moyens de chauffage qui sont entourés d'une gaine, de façon à former des éléments chauffants.

Lesdits éléments chauffants apportent la chaleur nécessaire au démarrage de la réaction de pyrolyse. Le nombre total desdits éléments, dans la zone de chauffage est fixé par l'utilisateur. II dépend essentiellement de la nature de la charge à convertir et de la taille du dispositif.

Les caractéristiques des éléments chauffants, leur nombre, la distance les séparant et leur configuration sont par exemple décrits dans les demandes de brevets de la demanderesse US 5 554 347 et EP 0 323 287. Lesdits éléments chauffants sont alimentés en énergie par tout moyen connu de l'homme du métier. Le plus souvent, ils sont alimentés en chauffage électrique ou au gaz, de préférence au gaz, soit isolément, soit en petits groupes, de telle sorte qu'ils définissent des sections de chauffage le long de la zone de chauffage. Ils peuvent ainsi moduler la quantité d'énergie fournie tout au long de cette zone. IIs permettent ainsi d'établir un profil thermique. La zone de chauffage est habituellement composée de 2 à 20 sections de chauffage, de préférence de 3 à 12 sections.

Ladite zone peut également comporter des moyens d'asservissement et de modulation de chauffage, comme ceux qui sont décrits, par exemple dans les demandes de brevet EP 0 323 287 et US 5 554 347.

Les moyens de chauffage peuvent être des résistances électriques entourées de gaines et chauffées par des électrodes, comme décrit dans les demandes de brevet EP 0 323 287 et US 5 160 501 de la demanderesse ou ils peuvent être constitués de gaines contenant un brûleur à gaz, comme décrit dans la demande de brevet US 5 554 347 de la demanderesse.

Les éléments chauffants forment des nappes qui sont sensiblement parallèles à l'axe de la zone de chauffage. On définit ensuite des rangées, chacune comprenant au moins une nappe d'éléments chauffants. Chaque rangée est sensiblement parallèle à l'axe de la zone de chauffage. Les rangées sont séparées par des cloisons non étanches en matériau céramique. Lesdites cloisons ont des formes adaptées, qui permettent de créer des zones de turbulences à l'intérieur des rangées, afin de favoriser la réaction de pyrolyse et de formation de méthylacétylène - propadiène.

Les gaines entourant les moyens de chauffage sont le plus souvent en matériau céramique. Elles peuvent être disposées de façon superposée ou en quinconce et peuvent former en projection transversale un faisceau à pas triangulaire, carré ou rectangulaire. Dans le cas de résistances électriques, la demande de brevet US 5 160 501 de la demanderesse montre qu'il est nullement nécessaire d'avoir une étanchéité parfaite au niveau des gaines, de façon à laisser diffuser au moins une partie d'un gaz de gaine G contenu dans l'espace formé par lesdites gaines et les résistances. Ce gaz G contient de l'hydrogène et/ou de la vapeur d'eau et/ou du monoxyde de carbone et/ou un gaz inerte, qui peut diffuser de l'intérieur vers l'extérieur des gaines sans perturber la réaction de pyrolyse. Il est dilué alors dans le mélange gazeux. Lesdites gaines sont également décrites dans les demandes de brevets précédemment citées.

Le temps de séjour total de la charge, dans la zone de chauffage est habituellement compris entre environ 12 et 2000 millisecondes (ms), de préférence entre environ 56 et 1500 ms et de manière encore plus préférée entre environ 111 et 1100 ms. La pression absolue est généralement supérieure à la pression atmosphérique. Le plus souvent supérieure, elle est supérieure à 1,1 bars (0,11 MPa), de préférence comprise entre environ 1,1 et 10 bars (0,11 et 1 MPa) et de manière encore plus préférée entre environ 1,2 et 5 bars (0,12 et 0,5 MPa).

Ladite zone de chauffage est formée d'une première zone de préchauffage de la chargé. Les moyens de chauffage sont chauffés de façon à ce que la température du mélange gazeux monte d'environ 500 à 1200°C par longueur de zone de chauffage. La température de sortie de ladite zone est la température minimale de pyrolyse de la charge.

La zone de pyrolyse permet de convertir au moins en partie la charge. Le temps de séjour de ladite charge dans la zone de pyrolyse, est habituellement d'environ 10 à 1000 ms, de préférence d'environ 50 à 900 ms, et de manière encore plus préférée d'environ 100 à 700 ms. L'énergie fournie aux sections de chauffage est modulée de façon à ce que la montée en température dans la zone de pyrolyse soit habituellement d'environ 200 à 500°C par longueur de zone de chauffage.

La zone de formation de méthylacétylène - propadiène opère à haute température. Le temps de séjour du mélange gazeux doit être court, pour éviter la formation de sous-produits. II dépend essentiellement de la nature de la charge à convertir. Il est généralement compris entre environ 1 et 400 ms, le plus souvent entre environ 5 et 300 ms et de manière encore plus préférée entre environ 10 et 200 ms. Il est en règle général, inférieur à celui de la zone de pyrolyse. On module l'énergie fournie aux différentes sections de chauffage, de façon à ce que la montée en température dans la zone formation de méthylacétylène - propadiène soit généralement comprise entre environ 700 et 1500°C par longueur de zone de chauffage. La température finale en sortie de la zone de chauffage est habituellement comprise entre environ 800 et 1300°C, de préférence entre environ 900 et 1100°C

La zone de chauffage est suivie d'une zone de refroidissement (ou trempe), de façon à abaisser rapidement la température des effluents obtenus à l'issue de la zone de chauffage. Dans le cas d'une trempe directe, les effluents sont rapidement mis en contact avec un fluide de refroidissement (agent de trempe), bien connu de l'homme du métier. Le fluide est généralement injecté dans les effluents au moyen d'injecteurs le plus souvent en matériau céramique, disposés en périphérie de la zone de chauffage et reliés à une source extérieure de fluide de trempe. L'ensemble des gaz effluents est recueilli par un orifice de sortie à l'extrémité de la zone de réaction.

Dans le cas d'une trempe indirecte, les effluents peuvent être au moins en partie refroidis en circulant à travers des conduits étanches disposés dans la zone de refroidissement par lesquels s'écoule l'agent de trempe, ces conduits étant reliés à la source extérieure de l'agent de trempe.

Le procédé selon l'invention est habituellement mis en oeuvre dans un dispositif comprenant un réacteur (1) de forme allongée selon un axe, de préférence à section carrée ou rectangulaire, comportant à une première extrémité des moyens d'alimentation (5) en mélange gazeux, renfermant au moins un hydrocarbure à au moins trois atomes de carbone, à l'extrémité opposée des moyens d'évacuation (8) des effluents produits et entre ces deux extrémités des moyens de chauffage puis des moyens de refroidissement. Ledit réacteur comprend au moins deux rangées sensiblement parallèles à l'axe du réacteur, séparées par une cloison (9) non étanche en matériau réfractaire entre ces deux rangées. Chaque rangée comporte une pluralité de moyens de chauffage (3), lesdits moyens étant disposés en nappes d'éléments chauffants et entourés de gaines (4) en matériau céramique sensiblement parallèles entre elles et sensiblement perpendiculaires à l'axe du réacteur, de façon à définir entre les gaines et/ou les nappes formées des espaces ou passages pour la circulation des mélanges gazeux et/ou des effluents. Lesdits moyens de chauffage et lesdites gaines sont adaptés à chauffer lesdits passages par sections transversales successives, indépendantes et sensiblement perpendiculaires à l'axe du réacteur. Ledit réacteur comporte des moyens d'asservissement et de modulation de chauffage reliés aux dits moyens de chauffage. Il comporte également des moyens de refroidissement (7) des effluents reliés audits moyens d'alimentation en fluide de refroidissement.

Dans une forme particulière du procédé selon l'invention, ledit réacteur peut être précédé d'un vapocraqueur. Ce dispositif est notamment décrit dans la demande de brevet FR 2 748 273 de la demanderesse. Il permet le décokage du réacteur en continu.

Le vapocraqueur comprend au moins deux tubes de vapocraquage, chaque tube étant connecté à une extrémité à une ligne d'alimentation en charge. Lesdites lignes d'alimentation sont contrôlées par des vannes de régulation. Ces vannes permettent la circulation du mélange gazeux (renfermant la charge hydrocarbonée et un fluide formé essentiellement de vapeur d'eau) dans certains tubes du vapocraqueur, ainsi que la circulation d'un fluide seul formé essentiellement de vapeur d'eau, dans d'autres tubes. Le mélange gazeux issu des tubes du vapocraqueur contenant les produits du craquagé, circule ensuite dans au moins une rangée du réacteur de pyrolyse, le fluide formé essentiellement de vapeur d'eau, dans au moins une autre rangée, de façon à réaliser le décokage. Les vannes permettent d'alterner les étapes de pyrolyse et de décokage, dans chaque rangée du réacteur de pyrolyse.

Concernant le refroidissement, les produits formés et l'effluent de décokage peuvent être mélangés avant d'être introduits dans la zone de refroidissement. Ils peuvent également être refroidis séparément dans leurs rangées respectives situées au niveau de la zone de refroidissement, puis éventuellement être mélangés.

L'invention sera mieux comprise par la description de différents modes de réalisation, donnés à titre d'exemple illustratif mais nullement limitatif.

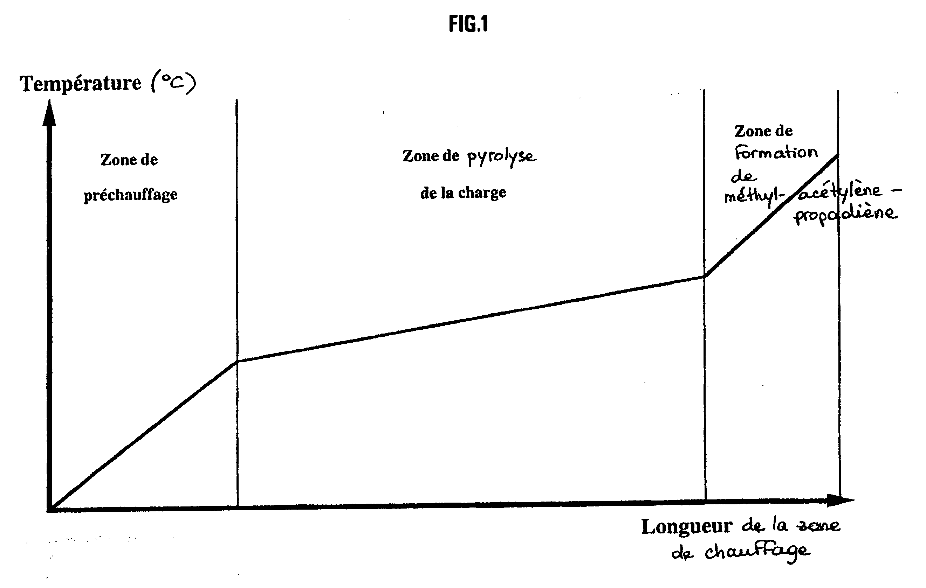

La figure 1 illustre un profil de température théorique, pour une charge quelconque, dans la zone de chauffage. Ledit profil est exprimé en fonction de la température (°C) et de la longueur de la zone de chauffage. La zone de chauffage est chauffée de façon à obtenir une montée contrôlée en température dans chacune des trois zones successives. La courbe idéale de variation de température, dans chaque zone, est représentée par une droite. L'inclinaison de chaque droite (la pente) dépend de la nature de la charge à craquer. Les moyens de chauffage permettent de réguler la montée en température dans chaque zone.

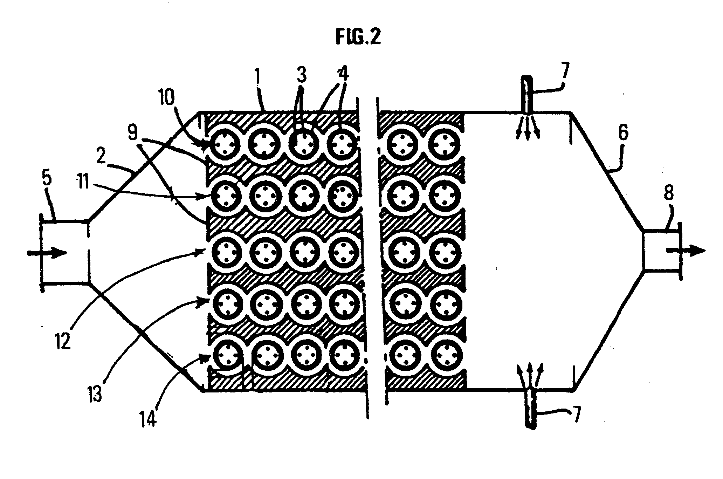

Sur la figure 2, on a représenté, selon un mode de réalisation, un réacteur (1) sensiblement horizontal, de forme allongée et de section rectangulaire, comprenant un distributeur (2) permettant d'alimenter par un orifice d'entrée (5) le réacteur en mélange gazeux réactionnel. Ce dernier, qui contient un mélange de vapeur d'eau et d'au moins un hydrocarbure ayant au moins trois atomes de carbone dans sa molécule, a été préchauffé de manière conventionnelle, de préférence par convection, dans une zone non représentée sur la figure.

Le réacteur comporte des rangées (10 à 14) séparées les unes des autres par des cloisons (9), non étanches en matériau céramique, de forme comportant des alvéoles adaptées à favoriser des turbulences à l'intérieur de la rangée et donc à favoriser la réaction. Ces rangées sont sensiblement parallèles entre elles et sensiblement perpendiculaires à l'axe du réacteur (1) défini selon la direction d'écoulement de la charge.

Chaque rangée (10 à 14) comprend une pluralité de moyens de chauffage (3) entourés de gaines (4), disposées en nappes parallèles et formant dans un plan (plan de la figure) un faisceau à pas carré. Dans le cas d'un chauffage électrique, ces gaines peuvent contenir une pluralité de résistances électriques (3) baignant dans un gaz de gaine G.

Lesdites nappes définissent des sections de chauffage transversales sensiblement perpendiculaires à l'axe du réacteur (1).

L'alimentation des sections de chauffage n'est pas représentée sur la figure. Dans le cas d'un chauffage électrique, il s'agit de paires d'électrodes. Dans le cas d'un chauffage au gaz, l'alimentation des sections s'effectue par la circulation d'un gaz ou d'un mélange de gaz. Des sondes pyrométriques assurent dans les deux cas la régulation automatique de la température de chaque section de chauffage, par un dispositif classique de régulation non représenté sur la figure.

Dans la première partie de la zone de chauffage, les moyens de chauffage sont chauffés de façon à ce que la température monte d'environ 500 à 1200°C par longueur de zone de chauffage. Le mélange gazeux est préchauffé, de façon à ce que sa température soit amenée à la température minimale de pyrolyse de la charge.

Le mélange gazeux circule ensuite dans la zone de pyrolyse. On module les sections de chauffage, de façon à ce que la température monte d'environ 200 à 500 °C par longueur de zone de chauffage.

La charge craquée parvient ensuite à la zone de formation de méthylacétylène - propadiène, où la température monte d'environ 700 à 1500°C par longueur de zone de chauffage.

Les effluents sont refroidis dans une zone de refroidissement (6). Ils sont mis en contact avec un agent de trempe introduit par l'intermédiaire d'injecteurs (7) de trempe disposés à la périphérie du réacteur (1) et reliés à une source extérieure de l'agent de trempe, non représentée sur la figure. L'ensemble des gaz effluents est refroidi à 500°C puis recueilli par un orifice de sortie (8) à l'extrémité du réacteur (1).

### Exemple 1 :

On utilise un réacteur horizontal à trempe indirecte, dont les moyens de chauffage sont des résistances électriques en carbure de silicium du type Crusilite de chez KANTHAL. Ces résistances sont entourées de gaines en carbure de silicium fritté, disposées concentriquement par rapport au centre du cercle englobant les résistances.

Ces gaines au nombre de 9 sont disposées en ligne perpendiculairement au sens de circulation de la charge (verticalement). La longueur de chaque résistance est de 100 mm et son diamètre de 10 mm. Les gaines en céramique ont une longueur de 110 mm, un diamètre extérieur de 50 mm et un diamètre intérieur de 42 mm. La distance séparant deux gaines voisines ou une gaine et la paroi du réacteur en béton réfractaire est de 5 mm.

La température du gaz le long du réacteur est régulée thermiquement par l'intermédiaire de thermocouples disposés dans les espaces où circule la charge.

On utilise comme charge du propylène à 99,7 % de pureté dilué avec de l'eau dans un rapport pondéral vapeur d'eau/charge de 1. La charge est préchauffée de manière conventionnelle à 450 °C.

On impose le profil de température suivant :

| **Zones thermiques** | **Longueur de zone par rapport à la zone de chauffage (%)** | **Températures entrée/sortie zone (°C)** | **Montée en température (°C par longueur de zone de chauffage)** | **Temps de séjour (ms) et Pression absolue (MPa)** |
|---|---|---|---|---|
| Préchauffage | 20 | 450-670 | 1100 | 22 ms; 0,15 MPa |
| Pyrolyse | 60 | 670-860 | 316 | 55 ms; 0,15 MPa |
| Formation méthylacétylène/ propadiène | 20 | 860 à 1030 | 850 | 22 ms; 0,15 MPa |

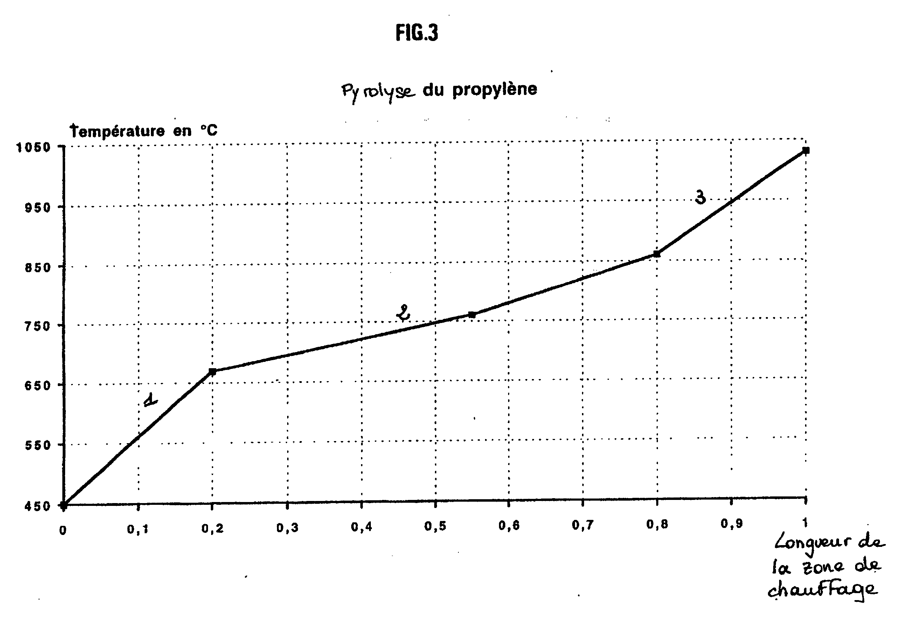

On a ainsi trois zones de montée en température, comme schématisé respectivement sur la figure 3 par les courbes 1, 2 et 3. La courbe 1 représente la montée en température dans la zone de préchauffage, la courbe 2, la montée en température dans la zone de pyrolyse et la courbe 3, la montée dans la zone de formation de méthylacétylène - propadiène.

Les gaz effluents sont refroidis rapidement dans un premier temps à 500 °C par échange indirect, puis d'autres échangeurs de température permettent ensuite d'abaisser leur température à la température ambiante.

Dans ces conditions, le taux de conversion du propylène est de 60,5 %.
Les sélectivités* des produits principaux sont les suivants:

| **Produits** | **Sélectivité (%)** |
|---|---|
| Méthane | 16,1 |
| Ethylène | 36,4 |
| Méthylacétylène | 8,2 |
| Propadiène | 12,4 |
| Acétylène | 3,2 |
| Benzène | 8,8 |

* La sélectivité du produit P est égale au nombre d'atomes de carbone du produit P multiplié par 100 et divisé par le nombre d'atomes de carbone de propylène converti.

### Exemple 2 :

On utilise un réacteur similaire à celui employé dans l'exemple 1 mais comprenant 21 éléments de chauffage.

La charge utilisée est du propane à 99,5 % de pureté, dilué avec de l'eau dans un rapport pondéral de vapeur d'eau/charge de 1. Elle est préchauffée de manière conventionnelle à 450°C.

On impose le profil de température suivant :

| **Zones thermiques** | **Longueur de zone par rapport à la zone de chauffage (%)** | **Températures entrée/sortie zone (°C)** | **Montée en température (°C par longueur de zone de chauffage)** | **Temps de séjour (ms) et Pression absolue (bar)** |
|---|---|---|---|---|
| Préchauffage | 24 | 450-670 | 916 | 147 ms; 1,5 bars |
| Pyrolyse | 57 | 670-860 | 339 | 353 ms; 1,5 bars |
| Formation méthylacétylène/ propadiène | 19 | 860-1050 | 1000 | 120 ms; 1,5 bars |

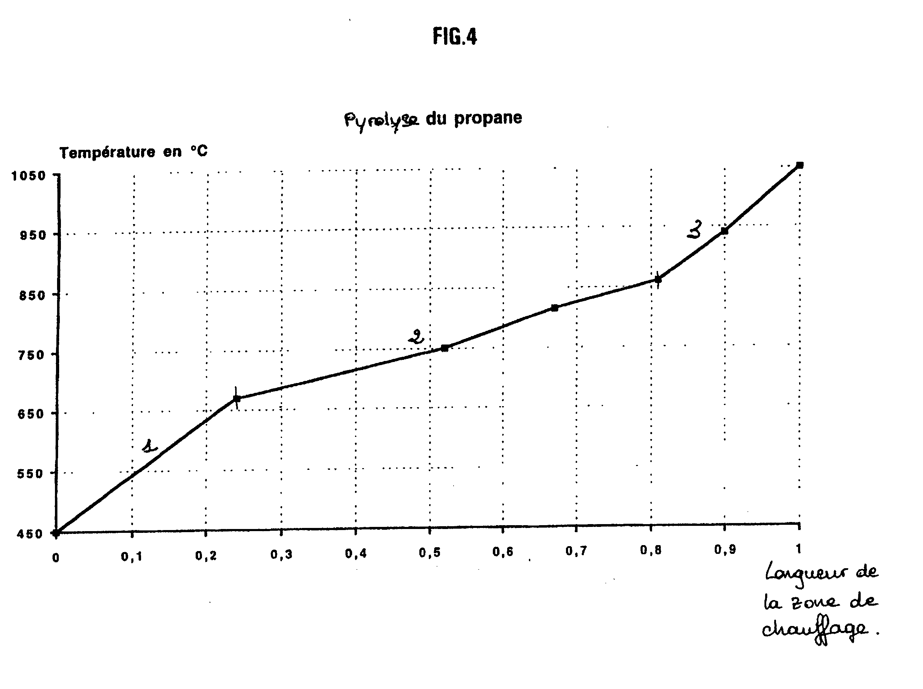

On a trois zones de montées en température, comme schématisé respectivement sur la figure 4 par les courbes 1, 2 et 3.

Dans ces conditions, le taux de conversion du propane est de 98,5%.

Les sélectivités des produits principaux sont les suivants :

| **Produits** | **Sélectivité (%)** |
|---|---|
| Méthane | 19,7 |
| Ethylène | 41,0 |
| Méthylacétylène | **6,6** |
| Propadiène | **9,9** |
| Acétylène | 3,1 |
| Benzène | 4,1 |
| Propylène | 8,3 |

## Revendications

1. Procédé de production de méthylacétylène et de propadiène dans une zone de réaction, de forme allongée selon une direction (un axe), comprenant une zone de chauffage et une zone de refroidissement, faisant suite à ladite zone de chauffage, dans lequel on fait circuler, dans la zone de chauffage un mélange gazeux renfermant au moins un hydrocarbure à au moins trois atomes de carbone et au moins un diluent, à une pression absolue supérieure à la pression atmosphérique, selon une direction d'écoulement sensiblement parallèle à la direction (à l'axe) de la zone de chauffage, ledit procédé étant **caractérisé en ce que** la zone de chauffage comprend au moins une zone de préchauffage dans laquelle la température dudit mélange gazeux augmente d'environ 500 à 1200°C par longueur de zone de chauffage, au moins une zone de pyrolyse de la charge dans laquelle la température monte d'environ 200 à 500°C par longueur de zone de chauffage et au moins une zone de formation de méthylacétylène - propadiène dans laquelle la température monte d'environ 700 à 1500°C par longueur de zone de chauffage,
**en ce que** le temps de séjour de la charge dans la zone de pyrolyse est compris entre environ 10 à 1000 millisecondes, le temps de séjour de la charge dans la zone de formation de méthylacétylène - propadiène est compris entre environ 1 et 400 millisecondes, le temps de séjour total de la charge dans la zone de chauffage est compris entre environ 12 et 2000 millisecondes,
les produits formés à l'extrémité de la zone de chauffage étant refroidis dans la zone de refroidissement puis recueillis à l'extrémité de la zone réactionnelle.

2. Procédé selon la revendication 1 dans lequel le diluent est choisi dans le groupe formé par la vapeur d'eau et l'azote.

3. Procédé selon la revendication 1 ou 2 dans lequel le rapport pondéral du diluent à la charge hydrocarbonée est compris entre environ 0,1:1 et 5:1.

4. Procédé selon l'une des revendications 1 à 3 dans lequel la température finale en sortie de la zone de chauffage est comprise entre environ 800 et 1300°C.

5. Procédé selon l'une des revendications 1 à 4 dans lequel la pression absolue est supérieure à 1,1 bar (0,11 MPa),

6. Procédé selon l'une des revendications 1 à 5 dans lequel le temps de séjour dans la zone de formation de méthylacétylène - propadiène est inférieur au temps de séjour dans la zone de pyrolyse.

7. Procédé selon l'une des revendications 1 à 6 dans lequel la zone de chauffage comporte au moins deux rangées sensiblement parallèles à l'axe séparées par une cloison non étanche en matériau réfractaire entre deux rangées successives, chaque rangée comprenant une pluralité de moyens de chauffage disposés en au moins une nappe d'éléments chauffants et entourés de gaines en matériau céramique sensiblement parallèles entre elles et sensiblement perpendiculaires à l'axe de la zone de chauffage.

## Patentansprüche

1. Verfahren zur Herstellung von Methylacetylen und Propadien in einer Reaktionszone mit einer gemäß einer Richtung (einer Achse) länglichen Form, umfassend eine Heizzone und eine Kühlzone . welche der Heizzone folgt, in welchem man in der Heizzone ein Gasgemisch zirkulieren lässt, das wenigstens einen Kohlenwasserstoff mit wenigstens drei Kohlenstoffatomen und wenigstens ein Verdünnungsmittel einschließt, bei einem Absolutdruck größer als dem Atmosphärendruck, gemäß einer Fließrichtung, die im wesentlichen parallel zur Richtung (zur Achse) der Heizzone ist, wobei das Verfahren **dadurch gekennzeichnet ist, dass** die Heizzone wenigstens eine Vor-Heizzone umfasst, in der die Temperatur des Gasgemischs sich auf etwa 500 - 1200 °C über die Länge der Heizzone erhöht, wenigstens eine Zone zur Pyrolyse der Charge, in der die Temperatur auf etwa 200 - 500 °C über die Länge der Heizzone ansteigt, und wenigstens eine Zone zur Bildung von Methylacetylen - Propadien, in der die Temperatur um etwa 700 - 1500 °C über die Länge der Heizzone ansteigt, dadurch, dass die Verweilzeit der Charge in der Pyrolysezone zwischen etwa 10 und 1000 Millisekunden liegt, die Verweilzeit der Charge in der Zone zur Bildung von Methylacetylen - Propadien zwischen etwa 1 und 400 Millisekunden liegt, die Gesamtverweilzeit der Charge in der Heizzone zwischen etwa 12 und 2000 Millisekunden liegt, wobei die am Ende der Heizzone gebildeten Produkte in der Kühlzone gekühlt und dann am Ende der Reaktionszone gesammelt werden.

2. Verfahren nach Anspruch 1, bei dem das Verdünnungsmittel aus der durch Wasserdampf und Stickstoff gebildeten Gruppe gewählt wird.

3. Verfahren nach Anspruch 1 oder 2, bei dem das Gewichtsverhältnis des Verdünnungsmittels zur Kohlenwasserstoff-Charge zwischen etwa 0,1:1 und 5:1 liegt.

4. Verfahren nach einem der Ansprüche 1 bis 3, bei dem die Endtemperatur am Ausgang der Heizzone zwischen etwa 800 und 1300 °C liegt.

5. Verfahren nach einem der Ansprüche 1 bis 4, bei dem der Absolutdruck über 1,1 bar (0,11 MPa) liegt.

6. Verfahren nach einem der Ansprüche 1 bis 5, bei dem die Verweilzeit in der Zone zur Bildung von Methylacetylen - Propadien unter der Verweilzeit in der Pyrolyse-Zone liegt.

7. Verfahren nach einem der Ansprüche 1 bis 6, bei dem die Heizzone wenigstens zwei, im wesentlichen zur Achse parallele Reihen, getrennt durch eine dichte Trennwand aus feuerfestem Material zwischen zwei aufeinander folgenden Reihen umfasst, wobei jede Reihe eine Vielzahl von Heizmitteln umfasst, die in wenigstens einem Mantel von Heizelementen angeordnet sind und von Hüllen aus keramischem Material umgeben sind, welche im wesentlichen zueinander parallel und im wesentlichen senkrecht zur Achse der Heizzone liegen.

## Claims

1. A process for producing methylacetylene and propadiene in a reaction zone which is elongate in one direction (one axis), comprising a heating zone and a cooling zone following said heating zone, in which a gas mixture comprising at least one hydrocarbon containing at least three carbon atoms and at least one diluent is circulated in the heating zone, at an absolute pressure which is above atmospheric pressure, in a flow direction substantially parallel to the direction (to the axis) of the heating zone, said process being **characterized in that** the heating zone comprises at least one pre-heating zone in which the temperature of said gas mixture increases by about 500°C to 1200°C per length of the heating zone, at least one zone for pyrolysis of the feed in which the temperature rises by about 200°C to 500°C per length of the heating zone and at least one methylacetylene-propadiene formation zone in which the temperature rises by about 700°C to 1500°C per length of the heating zone,
in which the residence time for the feed in the pyrolysis zone is in the range about 10 to 1000 milliseconds (ms), the residence time for the feed in the methylacetylene-propadiene formation zone is in the range about 1 to 400 milliseconds (ms), the total residence time for the feed in the heating zone is in the range about 12 to 2000 milliseconds (ms),
the products formed at the end of the heating zone being cooled in the cooling zone then recovered at the end of the reaction zone.

2. A process according to claim 1, in which the diluent is selected from the group formed by steam and nitrogen.

3. A process according to claim 1 or claim 2, in which the weight ratio of the diluent to the hydrocarbon-containing feed is in the range about 0.1:1 to 5:1.

4. A process according to any one of claims 1 to 3, in which the final temperature at the outlet from the heating zone is in the range about 800°C to 1300°C.

5. A process according to any one of claims 1 to 4, in which the absolute pressure is more than 1.1 bars (0.11 MPa).

6. A process according to any one of claims 1 to 5, in which the residence time in the methylacetylene-propadiene formation zone is shorter than the residence time in the pyrolysis zone.

7. A process according to any one of claims 1 to 6, in which the heating zone comprises at least two banks substantially parallel to the axis separated by a non fluid tight partition of refractory material between two successive banks, each bank comprising a plurality of heating means disposed in at least one layer of heating elements and surrounded by sheaths of ceramic material which are substantially parallel to each other and are substantially perpendicular to the axis of the heating zone.
